(19) Europäisches Patentamt
European Patent Office
Office européen des brevets

(11) EP 1 498 108 A2

(12) **DEMANDE DE BREVET EUROPEEN**

(43) Date de publication:
**19.01.2005 Bulletin 2005/03**

(51) Int Cl.[7]: **A61K 7/09**

(21) Numéro de dépôt: **04291777.3**

(22) Date de dépôt: **12.07.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **16.07.2003 FR 0308674**

(71) Demandeur: **L'OREAL**
**75008 Paris (FR)**

(72) Inventeurs:
- **Rollat-Corvol, Isabelle**
  **75017 Paris (FR)**
- **Samain, Henri**
  **91570 Bievres (FR)**

(74) Mandataire: **Wattremez, Catherine et al**
**L'OREAL - D.I.P.I.**
**25-29 Quai Aulagnier**
**92600 Asnières (FR)**

(54) **Composition comprenant un polymère conducteur et un agent reducteur, procédé de déformation permanente la mettant en oeuvre**

(57) La présente invention concerne une composition comprenant, dans un milieu cosmétiquement acceptable, (a) au moins un agent réducteur, (b) au moins un polymère conducteur de préférence comprenant au moins une unité répétitive de type des anilines, pyrroles, thiophènes ou bisthiophènes, furanes, para-phénylène-sulfures, paraphénylène vinylènes, indoles, amides aromatiques, hydrazides aromatiques, azométhines aromatiques, esters aromatiques particuliers.

Elle concerne de plus un procédé de traitement des fibres kératiniques, notamment humaines, et plus particulièrement les cheveux, consistant à a) appliquer sur les fibres kératiniques, sèches ou humides, une composition selon l'invention, et on laisse pauser pendant une durée suffisante à la mise en forme, b) on rince les fibres et on applique une composition oxydante, pendant une durée suffisante à la fixation de la forme, c) on rince éventuellement les fibres, d) on lave les fibres et on les rince, e) on sèche ou on laisse sécher les fibres.
Elle a enfin pour objet l'utilisation de cette composition pour conférer un effet optique aux fibres kératiniques.

EP 1 498 108 A2

## Description

L'invention concerne une composition comprenant, dans un milieu cosmétiquement acceptable, au moins un polymère conducteur et au moins un agent réducteur. Elle concerne également un procédé de traitement de fibres kératiniques, plus particulièrement de déformation permanente, mettant en oeuvre la composition précitée. Elle a enfin pour objet l'utilisation d'une telle composition afin d'apporter un effet optique aux fibres kératiniques.

Parmi les traitements applicable aux fibres kératiniques, notamment humaines, et plus particulièrement aux cheveux, figure la mise en forme permanente.

Ce procédé consiste à mettre en oeuvre deux étapes, la première consistant à mettre en contact lesdites fibres avec une composition réductrice afin de réduire les ponts di-sulfures présents dans la fibre kératinique. De plus, avant, après, ou simultanément à la réduction de ces ponts di-sulfures, le cheveu est mis en forme de la manière souhaitée. Celle-ci peut consister à friser le cheveu ou bien à le lisser, le résultat dépendant du moyen employé pour la mise sous tension. Une fois cette première étape de réduction et de mise sous tension effectuée, une étape de fixation est nécessaire pour rétablir les ponts di-sulfures et ainsi stabiliser la forme donnée. Cette opération est effectuée habituellement en milieu oxydant.

Il est clair que ce type de procédé est relativement agressif envers la fibre kératinique et après un tel traitement, cette dernière est relativement abîmée, et d'un aspect plus ou moins terne.

Par ailleurs, il est fréquent de devoir mettre en oeuvre des étapes de mise en forme permanente et de coloration à peu d'intervalle de temps, voire immédiatement l'une après l'autre, l'opération de mise en forme étant réalisée en premier lieu.

Là encore, il apparaît clairement que la mise en oeuvre d'une étape de coloration ultérieure représente un risque supplémentaire de dégradation, car les procédés de coloration sont bien souvent mis en oeuvre en présence d'un agent oxydant, en milieu alcalin.

Il est toujours possible de traiter les fibres avec un agent apportant, par exemple, de la brillance aux fibres ; ledit agent pouvant soit être présent dans la composition de coloration même, soit apporté dans une composition appliquée après le traitement de coloration.

Toutefois, l'effet de brillance obtenu manque d'intensité et donne en général aux fibres un aspect artificiel.

En outre, de telles compositions présentent l'inconvénient d'apporter un toucher gras ou collant aux fibres.

Enfin, au cas où l'on souhaiterait effectuer une étape de coloration après le procédé de mise en forme, la présence des composés précités peut limiter la montée du colorant dans les fibres et par conséquent donner des colorations moins intenses.

La présente invention a donc pour but de proposer des compositions comprenant au moins un agent réducteur et au moins un polymère conducteur, qui apportent aux fibres kératiniques traitées, un effet optique particulier sans les inconvénients rencontrés avec les compositions classiques.

Par ailleurs, lorsque les polymères conducteurs absorbent dans le domaine du spectre visible, la composition selon l'invention permet de colorer les fibres sans qu'un traitement particulier de coloration, postérieur au traitement de mise en forme des fibres, soit nécessaire.

Enfin, les fibres kératiniques présentent de manière avantageuse un toucher doux et agréable.

La présente invention a ainsi pour premier objet une composition comprenant, dans un milieu cosmétiquement acceptable,

(a) au moins un agent réducteur,
(b) au moins un polymère conducteur.

L'invention concerne de plus un procédé de traitement des fibres kératiniques, notamment humaines, et plus particulièrement des cheveux, consistant à mettre en oeuvre les étapes suivantes :

a) on applique sur les fibres kératiniques, sèches ou humides, une composition selon l'invention, et on laisse pauser pendant une durée suffisante à la mise en forme,
b) on rince éventuellement les fibres et on applique éventuellement une composition oxydante, pendant une durée suffisante à la fixation de la forme,
c) on rince éventuellement les fibres,
d) on lave éventuellement les fibres et on les rince,
e) on sèche ou on laisse sécher les fibres.

L'invention a de même pour objet l'utilisation d'une composition comprenant au moins un agent réducteur et au moins un polymère conducteur pour conférer un effet optique aux fibres kératiniques.

En effet, la composition selon l'invention apporte à l'ensemble des fibres kératiniques, et de façon uniforme,

un effet optique particulier, et notamment une brillance substantiellement plus intense, plus naturelle, et plus esthétique qu'avec les moyens de l'art antérieur.

Par ailleurs, lorsque les polymères conducteurs présents dans la composition selon l'invention absorbent dans le spectre visible, on obtient simultanément un effet optique, comme de la brillance, et de la couleur.

Dans un tel cas, la mise en oeuvre d'un procédé ultérieur de coloration peut ne plus être nécessaire.

Mais d'autres caractéristiques et avantages de l'invention apparaîtront plus clairement à la lecture de la description et des exemples qui suivent.

Dans ce qui va suivre et à moins d'une indication différente, les bornes d'un domaine de valeurs sont comprises comme faisant partie de ce domaine.

Au sens de la présente invention, le terme effet optique recouvre des effets de brillance, de couleur, métallique, goniochromatique, moiré.

Par ailleurs, et plus particulièrement, il est à noter que la brillance correspond à l'intensité lumineuse réfléchie sous un angle $\alpha$ lorsque la mèche de cheveux est éclairée sous un angle $-\alpha$. L'angle $\alpha$ classiquement utilisé pour mesurer cette réflexion spéculaire, autrement dit la brillance, est égal à 20°. Cet apport de brillance peut être mesuré par utilisation d'un brillancemètre comme il est par exemple décrit dans la norme ISO 2813 - 1994 de l'AFNOR (août 1994, rectificatif février 1997).

Polymères conducteurs

Selon la présente invention, on entend par "polymère conducteur" une structure moléculaire dans laquelle le ou les monomères présentent une forte délocalisation électronique et dont la disposition dans le squelette du polymère permet aux orbitales $\pi$ de se recouvrir. Cette caractéristique chimique se traduit par un phénomène de conduction électrique qui s'accompagne ou non d'un phénomène d'absorption dans le spectre UV-visible, voire dans l'infrarouge.

Par polymère conducteur absorbant dans le visible, on entend au sens de la présente invention, tout polymère conducteur présentant une absorbance non nulle dans le domaine de longueur d'ondes allant de 400 à 800 nm, même si les maxima d'absorption du polymère se situent en dehors de cette gamme.

Les polymères conducteurs mis en oeuvre dans le cadre de la présente invention sont des polymères conducteurs solubles ou dispersibles dans le milieu cosmétique approprié à l'application.

On dit que le polymère est soluble dans le milieu lorsqu'il forme un liquide limpide isotrope à 25°C dans le milieu comprenant de l'eau ou un mélange eau/solvant ; ceci étant obtenu dans tout ou partie d'une gamme de concentration comprise entre 0,01 et 50 % en poids de polymère conducteur.

De façon préférée, les polymères conducteurs mis en oeuvre dans le cadre de la présente invention sont des polymères conducteurs solubles ou dispersibles dans un milieu aqueux, avantageusement dans l'eau.

On dit que le polymère est dispersible dans le milieu comprenant de l'eau ou un mélange eau/solvant si, à 0,01% en poids, à 25°C, il forme une suspension stable de fines particules, généralement sphériques. La taille moyenne des particules constituant ladite dispersion est inférieure à 1 $\mu$m et, plus généralement, varie entre 5 et 400 nm, de préférence de 10 à 250 nm. Ces tailles de particules sont mesurées par diffusion de lumière.

Il est à noter que de manière avantageuse, ces polymères ne nécessitent pas l'emploi d'un agent dispersant.

De préférence, les polymères conducteurs se présentent sous une forme soluble dans le milieu de la composition.

De plus, les polymères présentent de manière avantageuse, une conductivité comprise entre $10^{-5}$ et $5.10^{5}$ siemens/cm, plus particulièrement comprise entre $10^{-3}$ et $10^{5}$ siemens/cm, et de préférence comprise entre $10^{-1}$ et $10^{4}$ siemens/cm.

La conductivité est mesurée à l'aide d'un générateur de courant (RM2 Test Unit commercialisé par la société Jandel) muni d'une tête de mesure dite quatre pointes (Universal four-point probes commercialisé par la société Jandel). Les quatre pointes alignées et distantes du même espacement d sont appliquées par simple pression sur l'échantillon à analyser. Un courant I est injecté par les pointes externes à l'aide de la source de courant créant ainsi une variation de potentiel. La tension U est mesurée entre les deux pointes internes reliées au voltmètre du générateur de courant.

Dans cette configuration la conductivité de l'échantillon exprimée en S/cm est donnée par l'expression suivante :

$$\sigma = (K \times I) / (U \times e)$$

Avec :

K coefficient dépendant de la position des contacts sur la surface de l'échantillon.
Lorsque les pointes sont alignées et équidistantes, K est égal à : $\Pi/\log(2)$

I : valeur du courant injecté exprimé en ampères
U : valeur de la tension mesurée exprimée en volts
e : épaisseur de l'échantillon exprimée en cm

Cette expression ne peut être utilisée que lorsque l'épaisseur du matériau est négligeable devant la distance d existant entre deux pointes (e/d < 0,25). Pour obtenir des épaisseurs suffisamment faibles et ainsi pouvoir calculer la conductivité du matériau, il est préconisé de réaliser la mesure sur un support non conducteur (par exemple, une lame de verre) recouvert du matériau à analyser obtenu par évaporation d'une solution diluée. Afin d'améliorer l'homogénéité du revêtement à analyser, il est également préconisé d'utiliser la technique de dépôt dite du spin coating.

Selon un mode de réalisation particulier, les polymères conducteurs présents dans la composition sont choisis parmi les polymères comprenant au moins une unité répétitive de formules suivantes :

<u>les anilines de structure (I) suivante</u> :

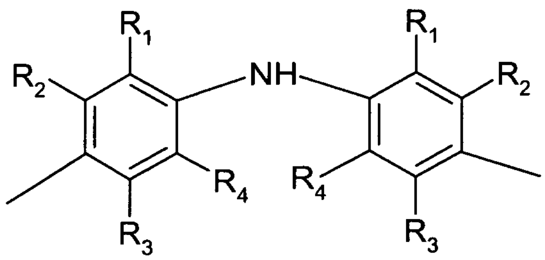

(I)

<u>les pyrroles de structure (IIa) et (IIb) suivantes</u> :

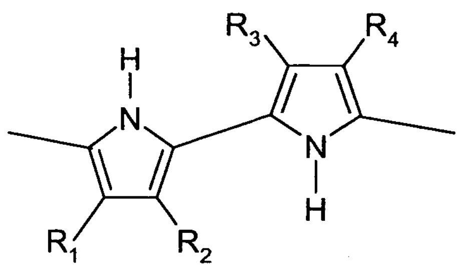

(IIa)

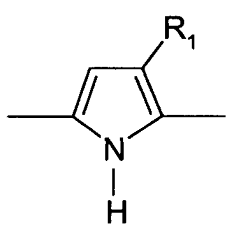

(IIb)

<u>les thiophènes ou bisthiophènes de formules (IIIa), (IIIb) et (IIIc) suivantes</u> :

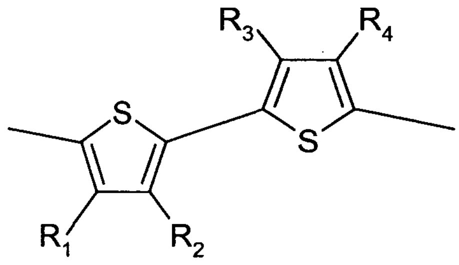

(IIIa)

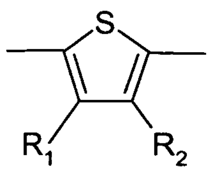

(IIIb)

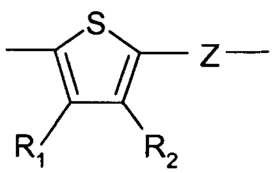

(IIIc)

les furanes de formule (IV) suivante :

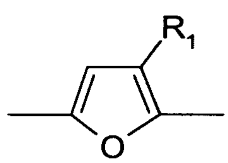

(IV)

les para-phénylène-sulfure de structure (V) suivante :

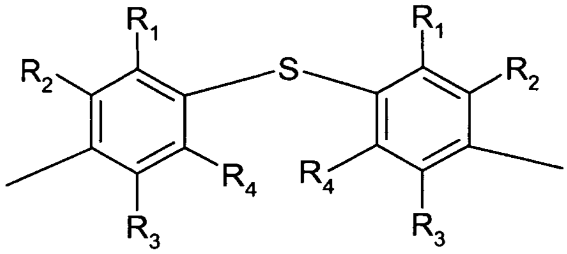

(V)

les paraphénylène vinylène de formule (VI) suivante :

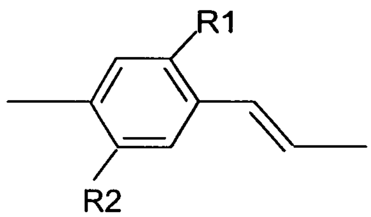

(VI)

les indoles de formule (VII) suivante :

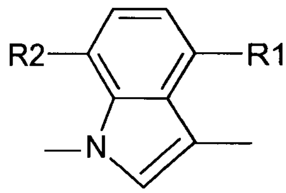

(VII)

les amides aromatiques de formules suivantes (VIIIa), (VIIIb), (VIIIc), (VIIId) :

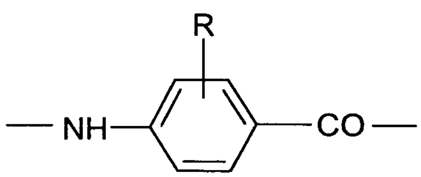

(VIIIa)

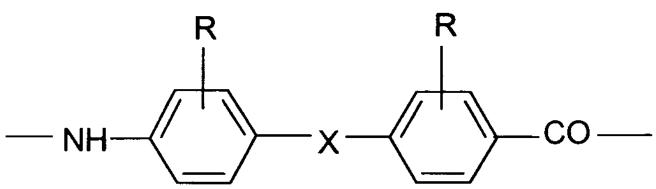

(VIIIb)

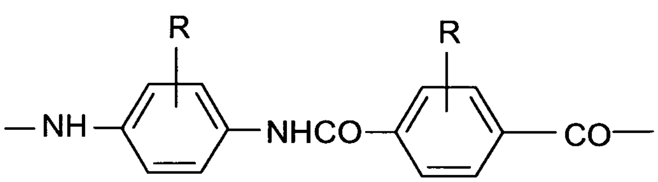

(VIIIc)

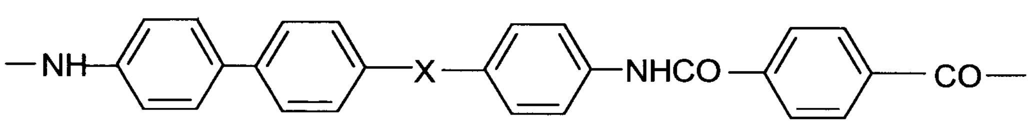

(VIIId)

les hydrazides aromatiques de formules (IXa), (IXb) et (IXc) suivantes :

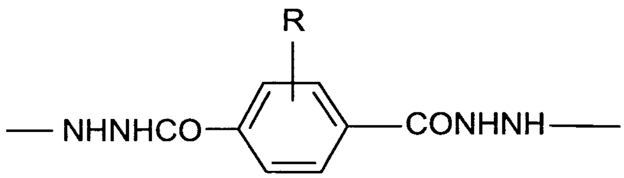

(IXa)

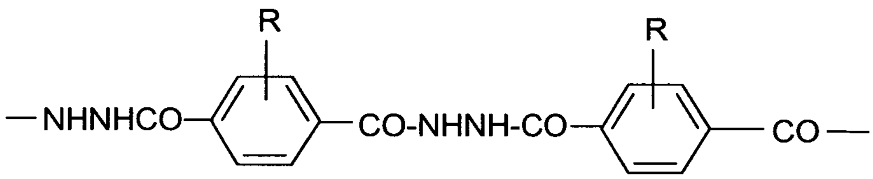

(IXb)

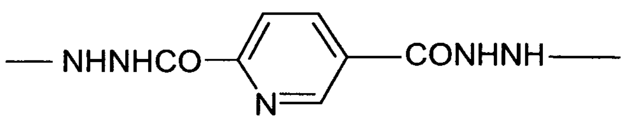

(IXc)

<u>les azométhines aromatiques de formules (Xa), (Xb) et (Xc) suivantes</u> :

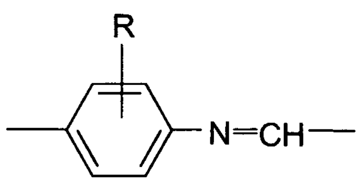

(Xa)

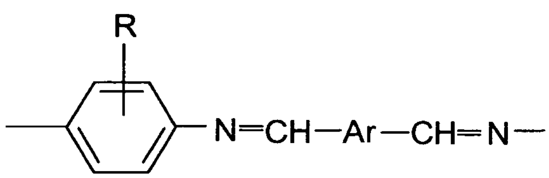

(Xb)

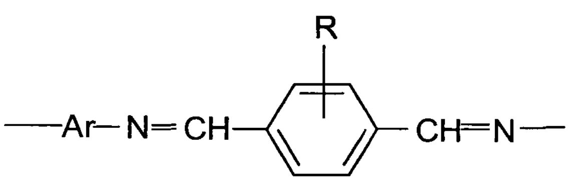

(Xc)

<u>les esters aromatiques de formules (XIa), (XIb) et (XIc) suivantes</u> :

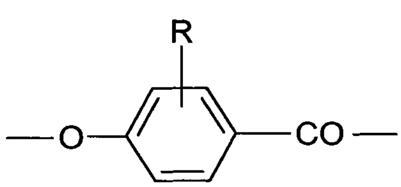

(XIa)

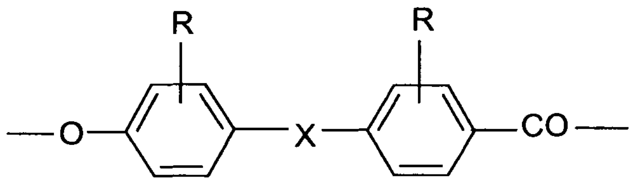

(XIb)

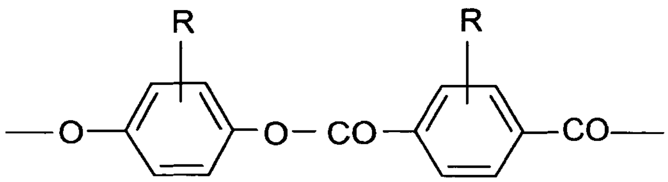

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et un groupement solubilisant ;
Ar représente un radical comprenant un radical monoaromatique ou polyaromatique X = -NHCO-, -O-, -S-, $-SO_2-$, -N=N-, $-C(CH_3)_2-$, $-CH_2-$, -CH=CH-, -CH=N- ;
Z = —CH=CH— ou —C≡C—.

Plus particulièrement, Ar représente au moins un radical choisi parmi les suivants :

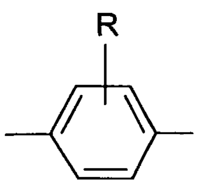
,
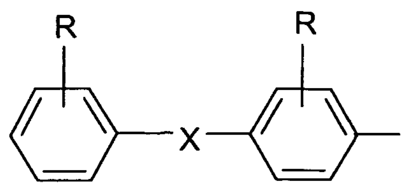
,
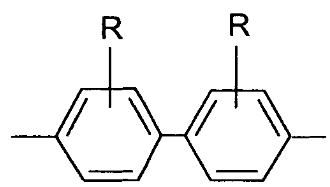
.

Par groupement solubilisant, on entend au sens de la présente invention, un groupement qui assure la solubilisation de ladite molécule dans le milieu cosmétique, de façon que le polymère présente un caractère conducteur après séchage de la composition.
Il est clair que le polymère conducteur présent dans la composition selon l'invention peut comprendre une ou plusieurs unités répétitives comprenant un ou plusieurs groupements solubilisants, et d'une ou plusieurs autres qui en sont dépourvues.
Les groupements solubilisants sont de préférence choisis dans le groupe formé par :

- un radical carboxylique (-COOH), carboxylate (-COO-M+ avec M représentant un métal alcalin comme le sodium, le potassium, un métal alcalino-terreux, une amine organique telle que une amine primaire, secondaire ou tertiaire, une alcanolamine, un acide aminé),
- un radical sulfonique ($-SO_3H$), sulfonate ($-SO_3^- M^+$, M ayant la même définition que ci-dessus),
- un radical amine primaire, secondaire, tertiaire,
- un radical ammonium quaternaire tel $-NR'3^+ Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- un radical hydroxyle,
- un radical polyoxyde d'alkylène en $C_2$-$C_3$.

Les fonctions acides carboxyliques ou sulfoniques peuvent ou non être neutralisées par une base, telle que l'hydroxyde de sodium, l'amino-2 méthyl-2 propanol, la triéthylamine ou encore la tributylamine, par exemple.
Les radicaux amines peuvent ou non être neutralisés par un acide minéral, tel que l'acide chlorhydrique, ou par un

acide organique, tel que les acides acétique ou lactique, par exemple.

En outre, il est à noter que lesdits radicaux solubilisants peuvent être reliés au cycle par l'intermédiaire d'un groupement espaceur tel que par exemple un radical -R''-, -OR''-, - OCOR''- ou encore -COOR''- avec R'' représentant un radical alkyle, linéaire ou ramifié en $C_1$-$C_{20}$, comprenant éventuellement un ou plusieurs hétéroatomes, tels que l'oxygène par exemple.

De préférence les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : - COOH, $-CH_2COOH$, $-CH_2OH$, $-(CH_2)_6OH$, $-(CH_2)_3SO_3H$, $-O(CH_2)_3SO_3H$, - O $(CH_2)_3N(CH_2CH_3)_2$, $-[(CH_2)_2O]_xCH_2CH_2OH$, $-[(CH_2)_2O]_xCH_2CH_2OCH_3$ avec x nombre moyen compris entre 0 et 200.

Le nombre d'unités répétitives du polymère n varie habituellement de 5 à 10000, notamment de 5 à 1000, plus particulièrement de 10 à 1000 et de préférence de 20 à 700.

Plus particulièrement, le polymère conducteur est tel qu'au moins un radical parmi R, R1 à R4 désigne un groupement solubilisant.

Conformément à un mode de réalisation particulier de l'invention, le polymère conducteur mis en oeuvre comporte au moins un groupement solubilisant par unité répétitive. Ainsi, de préférence, au moins un radical parmi R, $R_1$ à $R_4$ désigne un groupement solubilisant.

De préférence, le polymère conducteur est soluble dans le milieu de la composition.

Les polymères conducteurs présents dans la composition selon l'invention sont bien connus de l'homme de l'art et décrits notamment dans l'ouvrage "Handbook of organic conductive molecules and polymers" - Wiley 1997- New York, Vol 1, 2, 3, mais aussi dans la revue Can. J. Chem. Vol 64, 1986.

Les polythiophènes et leur synthèse sont plus particulièrement décrits dans l'article tiré de la revue Chem. Mater. 1998, Vol.10, N°7 pages 1990-1999 - par les auteurs RASMUSSEN S.C., PICKENS J.C. et HUTCHISON J.E. "A new, general approach to tuning the properties of functionalized polythiophenes : The oxidative polymerization of monosubstituted bithiophenes" ; dans l'article tiré de la revue Macromolecules 1998, 31, pages 933-936, par les mêmes auteurs "Highly conjugated, water-soluble polymers via direct oxidative polymerization of monosubstituted bithiophenes". Outre la polymérisation par oxydation chimique ou électrochimique, ils peuvent être aussi obtenus par polycondensation (thiophène dihalogéné ; catalyse avec des complexes de nickel ou de palladium) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B) ou de type A-B (réaction de plusieurs monomères de type A-X-B) ; par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.

Les polymères conducteurs présents dans la composition selon l'invention sont par ailleurs décrits dans la demande internationale WO 99/47570.

Parmi les polymères conducteurs convenables à la réalisation de la présente invention, on peut citer plus particulièrement les polymères correspondant aux formules (IIIa), (IIIb) et (IIIc) dans lesquelles les groupes solubilisants sont de préférence un groupement acide carboxylique ; un groupement acide sulfonique ; un radical amine tertiaire ; un radical ammonium quaternaire tel que $-NR'_3{}^+ Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux ; lesdits groupes étant éventuellement reliés au cycle par un espaceur. Les fonctions acides carboxyliques ou sulfoniques peuvent ou non être neutralisées.

Ainsi, la polymérisation peut être réalisée par oxydation chimique ou électrochimique du monomère thiophène correspondant ou bien encore par polycondensation.

A titre d'illustration, les polythiophènes de formules (IIIa) et (IIIb) peuvent être obtenus par polymérisation par oxydation (par exemple avec une catalyse $FeCl_3$); par polycondensation de thiophène dihalogéné catalysée par des complexes de nickel ou de palladium (ex. : $NiCl_2(dppe)_2$) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B ) ou de type A-B (réaction de plusieurs monomères de type A-X-B)) ; par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.

Les polythiophènes vinylènes de formule (IIIc) avec Z représentant —CH=CH-, peuvent notamment être obtenus par polymérisation de Gilch en présence d'une base forte (tertiobutylate de potassium), de 2,5 bis (bromoalkylène) thiophène ; par polymérisation par la méthode de Wessling via l'utilisation de précurseur à base de sels de sulfonium et pyrolyse ; par réaction de Wittig Wittig-Horner.

Les polythiophènes éthynylènes de formule (IIIc) avec Z représentant —C≡C— peuvent être obtenus par couplage de Heck-Sonogashira (de type AA-BB ou A-B ; formation de liaison carbone-carbone entre une fonction acétylénique terminale (ou acétylénique vraie) et une fonction bromo ou iodo catalysée par un complexe de palladium/

cuivre ($PdCl_2(PPh_3)_3$, CuI ou $Cu(Oac)_2$) en présence d'une base telle que la triéthylamine, diisopropylamine, piperidine etc...) ; par méthathèse d'alkynes en présence d'un complexe de molybdène (de $Mo(CO)_6$).

En général la fonctionnalisation des polythiophènes, en d'autres termes, l'apport du ou des groupes solubilisants ou non, est réalisée sur le monomère avant sa polymérisation.

Dans certains cas, l'obtention du groupe solubilisant est obtenu par suite de traitement de polymère. C'est notamment le cas de la fonction acide carboxylique qui peut être obtenue par hydrolyse de l'ester correspondant.

De préférence, les groupes solubilisants sont choisis parmi les groupements acide carboxylique ; acide sulfonique ; les radicaux amine tertiaire ; ammonium quaternaire tels que $-NR'_3{}^+ Z^-$ avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels.
Les fonctions acides carboxyliques ou sulfoniques peuvent ou non être neutralisées.

Selon un mode de réalisation particulier de l'invention, le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

Les polymères conducteurs sont généralement présents dans la composition dans des proportions d'au moins 0,001 % en poids, plus particulièrement d'au moins 0,01 % en poids, de préférence d'au moins 0,1 % en poids et de manière encore plus préférée, d'au moins 0,5 % en poids, par rapport au poids total de la composition. Par ailleurs, la teneur en polymère conducteur est avantageusement d'au plus 50 % en poids, plus particulièrement d'au plus 30 % en poids, de préférence d'au plus 20 % en poids et de manière encore plus préférée d'au plus 10 % en poids, par rapport au poids total de la composition.

Selon un mode de réalisation particulièrement avantageux de l'invention, la teneur en polymère conducteur est comprise entre 0,1 et 50% en poids, plus particulièrement entre 0,1 et 30% en poids, et de préférence entre 0,5 et 10 % en poids, par rapport au poids total de la composition.

Comme indiqué auparavant, la composition selon l'invention comprend, outre ledit polymère conducteur, au moins un agent réducteur.

Plus particulièrement, l'agent réducteur présent dans la composition selon l'invention, est choisi parmi les réducteurs soufrés, les réductones.

En ce qui concerne plus spécialement les agents réducteurs soufrés, ces derniers sont avantageusement choisis parmi les composés présentant au moins une fonction thiol, sulfure ou sulfite.

A titre d'exemples de tels agents réducteurs, on peut citer notamment l'acide thioglycolique, β-mercaptoéthanol, l'acide thiolactique, leurs sels de métal alcalin ou alcalino-terreux et leurs esters ; la cystéine, la cystéamine et leurs dérivés ; l'homocystéine et l'un de ses sels ; le mercaptoaldéhyde ; la pénicillamine ; la glutathione ; le thioglycolate ; les sulfites, bisulfites et hydrosulfites de métal alcalin, alcalino-terreux ou d'ammonium ; la cystine ; leurs mélanges.
Parmi les métaux alcalins, sont préférés le sodium, le potassium, parmi les métaux alcalino-terreux convient particulièrement le calcium. Quant aux groupements ammonium, ceux-ci peuvent ou non être substitués par un ou plusieurs radicaux alkyles, linéaires ou ramifiés, comprenant 1 à 4 atomes de carbone, éventuellement porteur(s) d'un groupement hydroxyle.

Pour ce qui a trait aux réductones, on peut mentionner tout particulièrement l'acide ascorbique, l'acide isoascorbique, leurs sels ou esters ; l'hydroxypropanedial, le 2,3-hydroxy-2-cyclopentène-1-one, l'acide alpha-cétoglutarique.

La teneur en agent réducteur est généralement comprise entre 1 et 30% en poids, et de préférence entre 5 et 20% en poids par rapport au poids de la composition réductrice.

Classiquement, le milieu de cette composition comprend de l'eau ou un mélange eau et solvant organique cosmétiquement acceptable.

Parmi les solvants organiques convenables, on peut citer plus particulièrement, les alcools en C1-C4, tels que l'alcool éthylique, l'alcool isopropylique, les alcools aromatiques comme l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.

Les solvants décrits ci-dessus, s'ils sont présents, représentent habituellement de 1 à 40 % en poids, plus préférentiellement de 5 à 30 % en poids, par rapport au poids total de la composition.

La composition réductrice peut aussi comprendre des additifs usuels tels que des agents tensioactifs non ioniques, anioniques, cationiques amphotères ou zwittérioniques et parmi ceux-ci, on peut citer les alkylsulfates, les

alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés, ainsi que d'autres tensioactifs non-ioniques du type hydroxypropyléther.

Lorsque la composition réductrice comprend ce type d'additif, sa teneur est de manière habituelle, de moins de 30% en poids, et de préférence comprise entre 0,5 et 10% en poids, par rapport au poids de la composition réductrice.

Le pH de la composition est généralement compris entre 3 et 12 environ, et de préférence entre 5 et 11 environ. Il peut être ajusté à la valeur désirée au moyen d'agents acidifiants ou alcalinisants.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalins on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante :

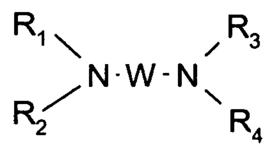
(A)

dans laquelle W est un reste propylène éventuellement substitué par un groupement hydroxyle ou un radical alkyle en $C_1$-$C_6$ ; $R_1$, $R_2$, $R_3$ et $R_4$, identiques ou différents, représentent un atome d'hydrogène, un radical alkyle en $C_1$-$C_6$ ou hydroxyalkyle en $C_1$-$C_6$.

La composition peut également comprendre divers adjuvants utilisés classiquement, tels que des polymères anioniques, cationiques, non ioniques, amphotères, zwittérioniques, des agents épaississants organiques ou minéraux, ainsi que des polymères associatifs non ioniques, anioniques ou amphotères ; des agents antioxydants, des agents de pénétration, des agents séquestrants, des parfums, des tampons, des agents dispersants, des agents de conditionnement, des agents filmogènes, des céramides, des agents conservateurs, des agents stabilisants, des agents opacifiants.

Lorsque les compositions sont destinées à une opération de lissage comme le défrisage ou le décrêpage des cheveux, la composition réductrice est de préférence sous forme d'une crème épaissie de façon à maintenir les cheveux aussi raides que possible pendant cette étape. On réalise ces crèmes sous forme d'émulsions "lourdes", obtenues par exemple en émulsionnant une phase aqueuse, comprenant notamment l'agent réducteur et le polymère conducteur, et une phase huileuse (huile végétale, huile de paraffine, esters d'acides gras, cire, par exemple).
On peut également utiliser des liquides ou des gels contenant des agents épaississants tels que des polymères ou des copolymères carboxyvinyliques qui "collent" les cheveux et les maintiennent dans la position lisse pendant le temps de pause.

Si l'opération est destinée à friser les fibres, ces dernières sont mises sous tension au moyen de bigoudis, avant, pendant ou après l'application de la composition selon l'invention.

Le temps de pause est en général compris entre 3 et 30 minutes, de préférence entre 5 et 15 minutes.

La composition oxydante mise en oeuvre lors de l'étape b) comprend de manière classique au moins un oxydant, en général de l'eau oxygénée, un bromate alcalin, un persel ou un polythionate.

La teneur en agent oxydant est comprise, plus particulièrement, entre 0,1% et 25 % en poids par rapport au poids de la composition oxydante.

Ladite composition peut aussi comprendre au moins un tensioactif, de préférence choisi parmi les composés non ioniques, anioniques ou amphotères. De manière avantageuse, on utilise des tensioactifs du type des alkylsulfates, des alkyléthersulfates, des bétaïnes, des dérivés d'imidazolium, des alkylpyrrolidones, des éthers d'alcools gras oxyalkylénés ou glycérolés, des esters d'acides gras de monoalcools ou polyol éventuellement oxyalkylénés ou glycérolés.

Plus particulièrement, lorsqu'ils sont présents, la teneur en tensioactif varie entre 0,01 et 30 % en poids par rapport au poids total de la composition, avantageusement, entre 0,1 et 20 % en poids et de préférence de 0,2 à 10% en poids du poids total de la composition.

La composition peut de plus comprendre un agent épaississant, et de préférence un système épaississant à base de polymères associatifs bien connus de l'homme de l'art et notamment de nature non ionique, anionique, cationique ou amphotère. A titre d'exemples, on peut citer tout particulièrement les homopolymères d'acide acrylique réticulés ; les homopolymères et copolymères réticulés d'acide 2-acrylamido-2-méthyl-propane sulfonique partiellement ou totalement neutralisés ; les homopolymères ou copolymères d'acrylate d'ammonium; les homopolymères ou

copoylmèers de diméthylaminoéthyl-méthacrylate quaternisé ; les gommes de guar non ioniques, de biopolysaccharides d'origine microbienne (scléroglucane, xanthane), issues d'exudats végétaux (arabique, Ghatti, Karaya, Tragacanthe) ; les hydroxypropyl ou carboxyméthyl celluloses ; les pectines; les alginates, ou leurs mélanges.

Si un tel agent est présent, sa teneur représente habituellement de 0,01 à 10% en poids, de préférence de 0,1 à 5% en poids, par rapport au poids total de la composition.

Il est de plus à noter que la composition peut comprendre un ou plusieurs agents de conditionnement tels que par exemple des cations, des silicones volatiles ou non, modifiées ou non, les huiles, les dérivés de polyuréthanes associatifs, les dérivés de celluloses associatifs, les dérivés de polyvinyllactames associatifs et les dérivés de polyacides insaturés associatifs, ou leurs mélanges.

Si un tel agent est présent, sa teneur représente habituellement de 0,0025 à 10% en poids, plus particulièrement 0,025 à 10% en poids, par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le pH de la composition oxydante est généralement compris entre 2 et 10.

Le procédé selon l'invention va maintenant être décrit.

Ainsi, il consiste à mettre en oeuvre les étapes :

a) on applique sur les fibres kératiniques, sèches ou humides, la composition selon l'invention, dont la description vient d'être donnée, et on laisse poser pendant une durée suffisante à la mise en forme,
b) on rince éventuellement les fibres et on applique éventuellement une composition oxydante, pendant une durée suffisante à la fixation de la forme,
c) on rince éventuellement les fibres,
d) on lave éventuellement les fibres et on les rince,
e) on sèche ou on laisse sécher les fibres.

Il est à noter que lors de l'étape a), les fibres kératiniques peuvent être mises en forme, en d'autres termes lissées ou mises sous tension au moyens de bigoudis, avant, après l'application de la composition selon l'invention, ou bien encore simultanément.

Le temps de pose est en général compris entre 3 et 30 minutes, de préférence entre 5 et 20 minutes.

Par ailleurs, et à titre purement indicatif, l'étape a) est habituellement réalisée à une température comprise entre 15 et 80°C, de préférence entre 20 et 40 °C.

Une fois l'étape a) effectuée, on rince les fibres, de préférence à l'eau, puis on applique la composition oxydante.

Il est bien évidemment important que la forme donnée aux fibres soit conservée durant cette étape.

Le temps de pose de la composition oxydante durant cette étape est en général comprise entre 3 et 30 minutes, de préférence entre 5 et 15 minutes.

En outre, la température à laquelle cette étape est mise en oeuvre est classiquement comprise entre 15 et 80°C, de préférence entre 20 et 40 °C.

Selon un mode de réalisation particulier, une fois l'étape b) effectuée, on rince les fibres imprégnées de la composition oxydante, généralement à l'eau.

Le cas échéant, on sépare, avant ou après le rinçage, la fibre kératinique des moyens nécessaires à sa mise sous tension.

Enfin, lors d'une étape d), on lave éventuellement les fibres traitées, de préférence avec un shampooing puis on les rince, habituellement à l'eau, puis lors d'une étape e), on sèche lesdites fibres ou bien encore on les laisse sécher.

Les exemples suivants illustrent l'invention sans en limiter la portée.

**EXEMPLE 1**

**Synthèse de poly(thiophène-3- acide acétique)**

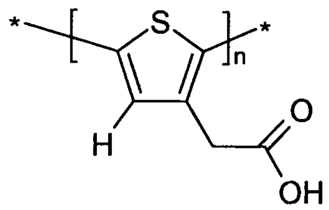

Mode opératoire

Préparation du polymère : poly(thiophène-3-acétate d'éthyle)

Dans un schlenk sous argon, on introduit 25 ml de chloroforme sec, on dégaze puis on introduit les réactifs : 2,5g de thiophène-3-acétate d'éthyle (14,7 mmol)

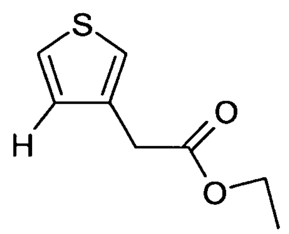

et 1 g de FeCl3 (6,15 mmol).
Le mélange est agité pendant 24 heures sous argon à 50°C.
Le polymère poly(thiophène-3-acétate d'éthyle) est alors précipité dans l'heptane.
Le polymère est ensuite dissous dans une solution de tétrahydrofuranne

Caractérisation par infra rouge :

Bande du C=O : 1719 $cm^{-1}$; bandes du CH2, CH3 = 2979 $cm^{-1}$, 2934 $cm^{-1}$ et disparition de la bande CH à 3102 $cm^{-1}$ présente dans le monomère.
**Hydrolyse du polymère** : poly(thiophène-3-acétate d'éthyle) pour former le poly(thiophène-3- acide acétique).
Le polymère obtenu précédemment est alors hydrolysé par un excès de 50 ml d'une solution aqueuse d'hydroxyde de sodium (2N) pendant 48heures à 70°C, suivi d'une acidification par HCl concentré jusqu'à précipitation du produit : poly(thiophène-3- acide acétique).
Le polymère est ensuite filtré et lavé à plusieurs reprises par de l'eau distillée afin d'éliminer les traces de catalyseur.

Caractérisation du polymère Infra-rouge :

Bande du C=O : 1740 cm-1 ; COO 1580 cm-1 ; OH (bande large 3000-3500 $cm^{-1}$)
Neutralisation du polymère poly(thiophène-3- acide acétique) :

Le polymère poly(thiophène-3- acide acétique) (2g) est dissous dans le tétrahydrofuranne (30g) et neutralisé à raison de 1 mol d'hydroxyde de sodium par mole d'acide carboxylique.
L'eau (30 g) est ensuite additionnée.
Le tétrahydrofuranne est évaporé.

Il est ainsi obtenu une solution aqueuse 6% de poly(thiophène-3- acide acétique) sous forme d'un sel de sodium.

| **Formulation 1 comprenant le polymère et procédé la mettant en oeuvre.** | |
|---|---|
| Poly(thiophène-3-acide acétique) | 5g |
| Acide thioglycolique | 3g |
| Monoéthanolamine | pH 8 |
| Alcool éthylique | 20 g |
| Eau qs | 100 g |

La formulation 1 est déposée sur cheveux foncés humides préalablement enroulés sur un rouleau de 9mm de diamètre. Après 20 minutes de pose, on applique une lotion oxydante contenant 8 volumes d'eau oxygénée amenée à pH 3 par de l'acide citrique. On laisse poser 5 minutes. Puis on déroule les cheveux et on procède au séchage (séchage libre).

<u>**EXEMPLE 2**</u>

| **Formulation 2 comprenant le polymère et procédé la mettant en oeuvre.** | |
|---|---|
| Poly(thiophène-3-acide acétique) | 5g |
| Cystéïne hydrochlorure | 7g |
| Monoéthanolamine | pH 9,4 |
| Alcool éthylique | 20 g |
| Eau qs | 100 g |

La formulation 2 est déposée sur cheveux foncés humides préalablement enroulés sur un rouleau de 9mm de diamètre. Après 30 minutes de pose à 45°C, on laisse poser 10 minutes à température ordinaire. On déroule et on laisse poser 15 minutes. Puis on procède au séchage (séchage libre).

**Revendications**

1. Composition comprenant, dans un milieu cosmétiquement acceptable,

   (a) au moins un agent réducteur,
   (b) au moins un polymère conducteur.

2. Composition selon la revendication précédente, **caractérisé en ce que** le polymère conducteur comprend au moins une unité répétitive de formules suivantes :

   <u>les anilines de structure (I) suivante</u> :

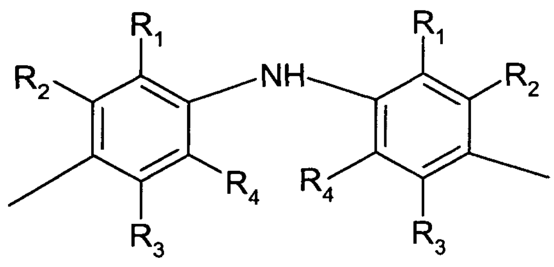

(I)

les pyrroles de structure (IIa) et (IIb) suivantes :

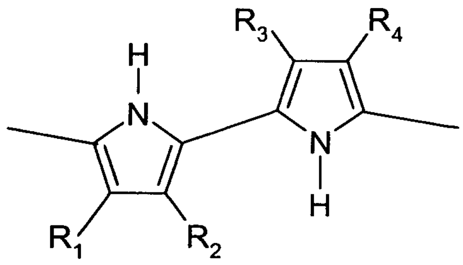

(IIa)

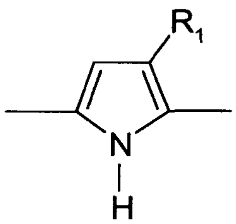

(IIb)

les thiophènes ou bisthiophènes de formules (IIa), (IIIb) et (IIIc) suivantes :

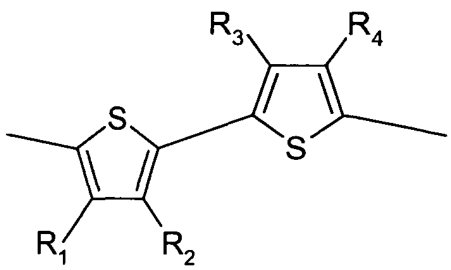

(IIIa)

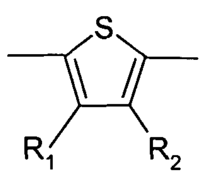

(IIIb)

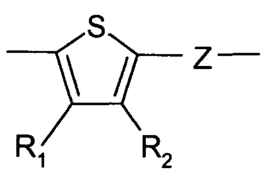

(IIIc)

les furanes de formule (IV) suivante :

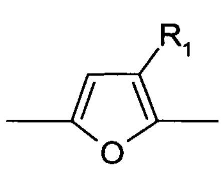

(IV)

les para-phénylène-sulfure de structure (V) suivante :

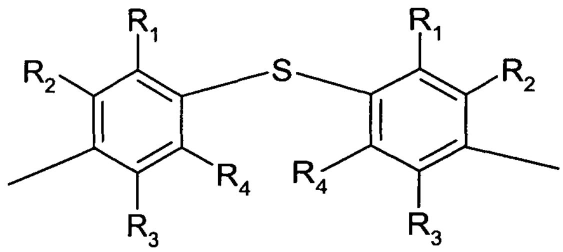

(V)

les paraphénylène vinylène de formule (VI) suivante :

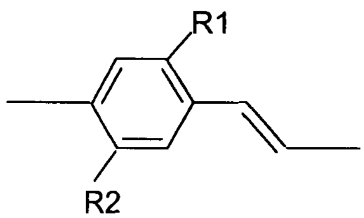

(VI)

les indoles de formule (VII) suivante :

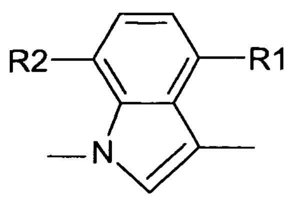

(VII)

les amides aromatiques de formules suivantes (VIIIa), (VIIIb), (VIIIc), (VIIId) :

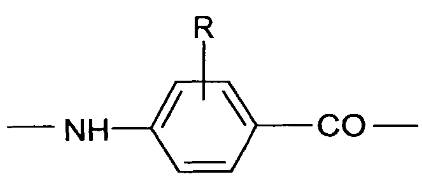

(VIIIa)

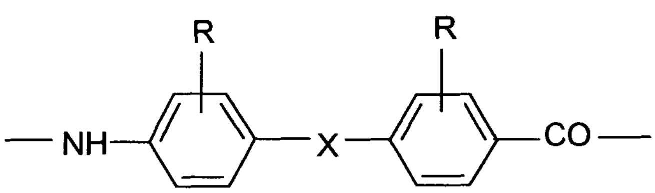

(VIIIb)

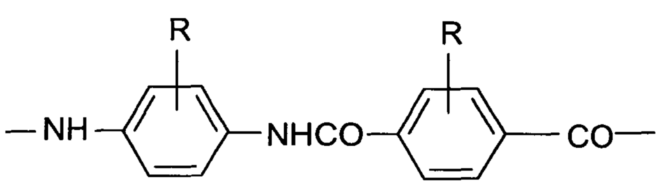

(VIIIc)

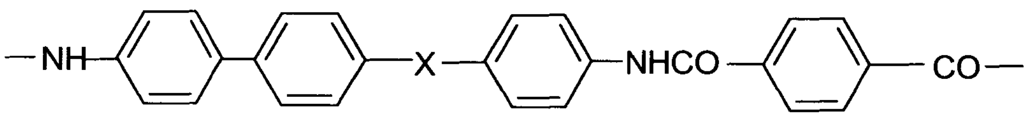

(VIIId)

les hydrazides aromatiques de formules (IXa), (IXb) et (IXc) suivantes :

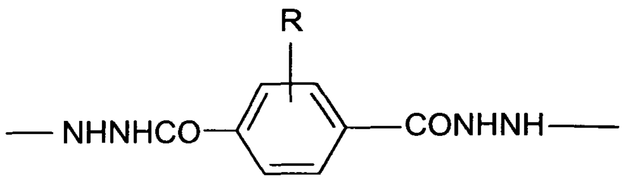

(IXa)

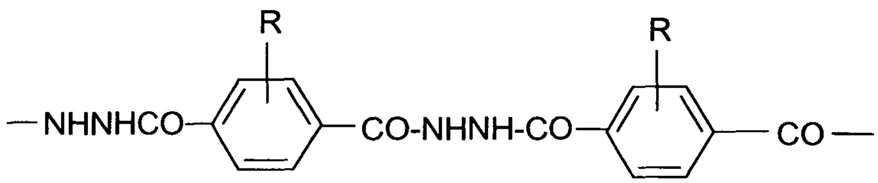

(IXb)

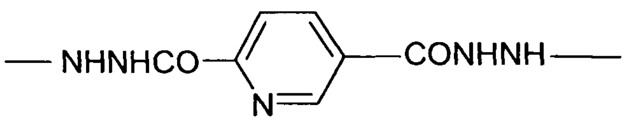

(IXc)

les azométhines aromatiques de formules (Xa), (Xb) et (Xc) suivantes :

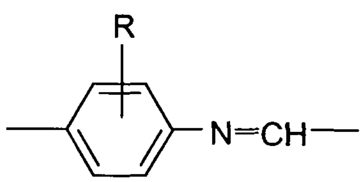

(Xa)

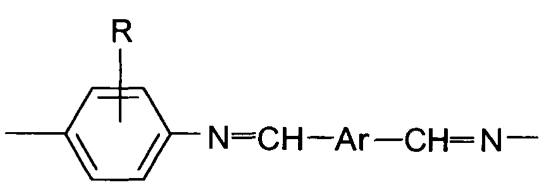

(Xb)

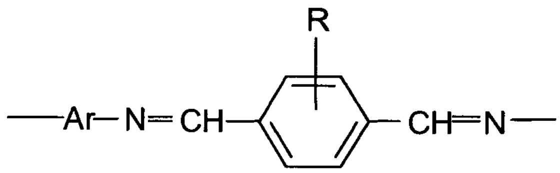

(Xc)

les esters aromatiques de formules (XIa), (XIb) et (XIc) suivantes :

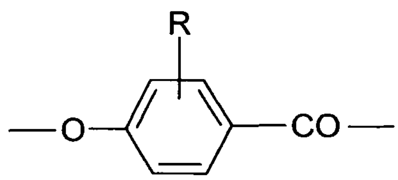

(XIa)

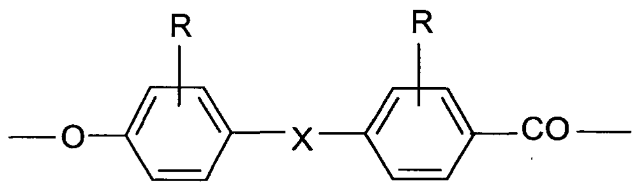

(XIb)

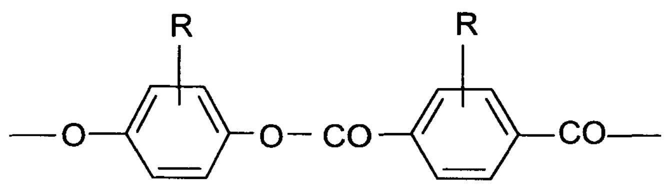

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et un groupement solubilisant ;
Ar représente un radical comprenant un radical monoaromatique ou polyaromatique X = -NHCO-, -O-, -S-, $-SO_2-$, -N=N-, $-C(CH_3)_2-$, $-CH_2-$, -CH=CH-, -CH=N- ;
Z = —CH=CH— ou —C≡C—.

3. Composition selon l'une des revendications 1 ou 2, **caractérisée en ce que** les groupements solubilisants sont choisis dans le groupe formé par :

- un radical carboxylique (-COOH), carboxylate ($-COO^-M^+$ avec M représentant un métal alcalin, un métal alcalino-terreux, une amine organique, une alcanolamine, un acide aminé),
- un radical sulfonique ($-SO_3H$), sulfonate ($-SO_3^-$ $M^+$, M ayant la même définition que ci-dessus),
- un radical amine primaire, secondaire, tertiaire,
- un radical ammonium quaternaire tel $-NR'_3{}^+$ $Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- un radical hydroxyle,
- un radical polyoxyde d'alkylène en $C_2$-$C_3$.

4. Composition selon l'une quelconque des revendications précédentes, **caractérisée en ce que** les groupements solubilisants sont reliés au cycle par l'intermédiaire d'un groupement espaceur tel que par exemple un radical -R"-, -OR"-, - OCOR"- ou -COOR"- avec R" représentant un radical alkyle, linéaire ou ramifié en $C_1$-$C_{20}$, comprenant

éventuellement un ou plusieurs hétéroatomes.

**5.** Composition selon l'une quelconque des revendications précédentes, **caractérisée en ce que** les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : -COOH, $-CH_2COOH$, $-CH_2OH$, $-(CH_2)_6OH$, $-(CH_2)_3SO_3H$, $-O(CH_2)_3SO_3H$, - $O(CH_2)_3N(CH_2CH_3)_2$, $-[(CH_2)_2O]_xCH_2CH_2OH$, $-[(CH_2)_2O]_xCH_2CH_2OCH_3$ avec x nombre moyen compris entre 0 et 200.

**6.** Composition selon l'une quelconque des revendications 1 à 3, **caractérisée en ce que** le polymère conducteur est tel qu'au moins un radical parmi R, R1 à R4 désigne un groupement solubilisant.

**7.** Composition selon l'une quelconque des revendications précédentes, **caractérisée en ce que** le polymère conducteur comporte au moins un groupement solubilisant par unité répétitive.

**8.** Composition selon l'une quelconque des revendications précédentes, **caractérisée en ce que** les groupements solubilisants sont choisis parmi les groupements acide carboxylique ; acide sulfonique ; les radicaux amine tertiaire ; ammonium quaternaire tels que $-NR'_3{}^+ Z^-$ avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels ; les fonctions acides carboxyliques ou sulfoniques peuvent ou non être neutralisées.

**9.** Composition selon l'une des revendications précédentes, **caractérisé en ce que** le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

**10.** Composition selon l'une quelconque des revendications précédentes, **caractérisée en ce que** le ou les polymères conducteurs sont présents dans des proportions d'au moins 0,001 % en poids par rapport au poids total de la composition.

**11.** Composition selon l'une quelconque des revendications précédentes, **caractérisée en ce que** le ou les polymères conducteurs sont présents dans des proportions d'au plus 50 % en poids par rapport au poids total de la composition.

**12.** Composition selon l'une quelconque des revendications précédentes, **caractérisée en ce que** le teneur en polymère conducteur représente 0,1 à 50 % en poids par rapport au poids total de la composition.

**13.** Composition selon l'une quelconque des revendications précédentes, **caractérisée en ce que** l'agent réducteur est choisi parmi les réducteurs soufrés, les réductones.

**14.** Composition selon la revendication précédente, **caractérisée en ce que** l'agent réducteur soufré est choisi parmi les composés présentant au moins une fonction thiol, sulfure ou sulfite.

**15.** Composition selon la revendication précédente, **caractérisée en ce que** l'agent réducteur soufré est choisi parmi l'acide thioglycolique, β-mercaptoethanol, l'acide thiolactique, leurs sels de métal alcalin ou alcalino-terreux et leurs esters ; la cystéine, la cystéamine et leurs dérivés ; l'homocystéine et l'un de ses sels ; le mercaptoaldéhyde ; la pénicillamine ; la glutathione ; le thioglycolate ; les sulfites, bisulfites et hydrosulfites de métal alcalin, alcalino-terreux ou d'ammonium ; leurs mélanges.

**16.** Composition selon la revendication 13, **caractérisée en ce que** l'agent réducteur de type réductone est choisi parmi l'acide ascorbique, l'acide isoascorbique, leurs sels ou esters ; l'hydroxypropanedial, le 2,3-hydroxy-2-cyclopentène-1-one, l'acide alpha-cétoglutarique.

**17.** Composition selon l'une quelconque des revendications 13 à 16, **caractérisée en ce que** la teneur en agent réducteur représente de 1 à 30 % en poids, par rapport au poids de la composition tinctoriale, de préférence de 5 à 20 % en poids, par rapport au poids de la composition tinctoriale.

**18.** Composition selon l'une quelconque des revendications précédentes, **caractérisée en ce que** le milieu cosmétiquement acceptable est l'eau ou un mélange eau-solvant.

**19.** Composition selon la revendication 18, **caractérisée en ce que** le ou les solvants sont choisis parmi des alcools en C1-C4, les alcools aromatiques, des glycols, des éthers de glycol, des polyols, des polyéthylèneglycols, le polypropylèneglycol et leurs mélanges.

**20.** Composition selon l'une quelconque des revendications précédentes, **caractérisée en ce qu'**elle comprend en outre au moins un agent tensioactif non ionique, anionique, cationique, amphotère ou zwittérionique.

**21.** Procédé de traitement des fibres kératiniques, notamment humaines, et plus particulièrement les cheveux, **caractérisé en ce qu'**il consiste à effectuer les étapes suivantes :

a) on applique sur les fibres kératiniques, sèches ou humides, une composition selon l'une quelconque des revendications précédentes, et on laisse pauser pendant une durée suffisante à la mise en forme,
b) on rince les fibres et on applique une composition oxydante, pendant une durée suffisante à la fixation de la forme,
c) on rince éventuellement les fibres,
d) on lave les fibres et on les rince,
e) on sèche ou on laisse sécher les fibres.

**22.** Utilisation d'une composition comprenant au moins un polymère conducteur, au moins un colorant direct, selon l'une quelconque des revendications 1 à 20, pour conférer un effet optique aux fibres kératiniques.

**23.** Utilisation selon la revendication précédente, **caractérisée en ce que** l'effet optique est de la brillance.